# EUROPEAN PATENT APPLICATION

(11) **EP 4 137 179 A1**
(43) Date of publication of application: **22.02.2023**
(21) Application number: 21788767.8
(22) Date of filing: 11.03.2021
(51) Int. Cl.: A61M 5/30, A61M 37/00, A61M 39/24

(54) **NEEDLE-FREE INJECTOR USING PULSE SHOCK WAVE**

(30) Priority: 14.04.2020 KR 20200045256
(71) Applicant: Jeisys Medical Inc., Geumcheon-gu Seoul 08501 (KR)
(72) Inventor: SEO, Kyu Young, Seoul 08501 (KR); YI, Won Ju, Seoul 08501 (KR); KANG, Dong Hwan, Incheon 22229 (KR)
(74) Representative: dompatent von Kreisler Selting Werner - Partnerschaft von Patent- und Rechtsanwälten mbB
(86) International application number: PCT/KR2021/003009
(87) International publication number: WO 2021/210785

(57) **Abstract**

The present invention relates to a needle-free injector using pulsed shock waves, the needle-free injector comprising: a power unit generating pulsed power; a pulsed shock wave generating unit which receives the pulsed power and generates pulsed shock waves; an upper housing in which a liquid and the pulsed shock wave generating unit are disposed; a lower housing which is connected to the upper housing, and in which a drug is disposed; a shock wave transmission unit which is provided between the upper housing and the lower housing to separate the upper housing and the lower housing; and an injection unit which is disposed in the lower housing and inject the drug.

## Description

### [TECHNICAL FIELD]

Embodiments of the inventive concept described herein relate to a needle-free injector using pulsed shock waves.

### [BACKGROUND ART]

A drug delivery system is a system designed to efficiently deliver a required amount of drug into the human body by minimizing side effects occurring in an existing method and maximizing therapeutic effects of the drug when using the drug for treatment of a disease or wound in the human body.

An injection method used most commonly in the drug delivery system enables accurate and efficient drug injection, but has problems such as injection phobia due to pain during injection, the risk of infection due to reuse, and generation of a large amount of medical waste.

In order to solve these problems, a drug delivery method such as a needle-free injector has been developed.

For example, a liquid injection technology, which is one of needle-free injection technologies, is a technology that applies shock waves to a liquid through a laser or electric waves to thermally expand the liquid and generate a high-speed liquid stream using the pressure generated at this time to inject the liquid into the skin.

However, the liquid injection technology has a problem in that the shock waves are generated in liquid, so that it is difficult to accurately adjust thermal conductivity (that is, the degree of expansion of the liquid) depending on the density, temperature, and type of the liquid. Furthermore, in the case of using a laser pulse having high energy and a short pulse width in order to generate the shock waves in the liquid, a laser device is required, and therefore the size and price of equipment may be increased. In addition, many optical systems are required to irradiate a laser beam to the liquid, which may result in problems such as damage to the optical systems.

### [DETAILED DESCRIPTION OF THE INVENTION]

### [TECHINICAL PROBLEM]

Embodiments of the inventive concept provide a needle-free injector using pulsed shock waves that easily adjusts the degree of expansion of a liquid, is implemented with small and economical equipment, and prevents damage to an optical system.

### [TECHNICAL SOLUTION]

According to an embodiment of the inventive concept, a needle-free injector using pulsed shock waves includes a power unit that generates pulsed power, a pulsed shock wave generating unit that receives the pulsed power and generates the pulsed shock waves, an upper housing in which a liquid and the pulsed shock wave generating unit are disposed therein, a lower housing, connected to the upper housing and that has a drug disposed therein, a shock wave transmitting unit provided between the upper housing and the lower housing and transmitting the shock waves generated in the upper housing to the lower housing, and an injection unit that is disposed in the lower housing and that injects the drug. The pulsed shock wave generating unit includes one or more shock wave generating electrodes that receive the pulsed power and allow a current to instantaneously flow, a shock wave generating unit that generates the pulsed shock waves as the current instantaneously flows between the one or more shock wave generating electrodes, and an insulating tube disposed in proximity to at least one of the shock wave generating electrodes in contact or non-contact with the at least one of the shock wave generating electrodes.

In addition, according to an embodiment, a needle-free injector using pulsed shock waves includes a power unit that operates a voltage charged in a capacitor to a switch and instantaneously generates pulsed power, a pulsed shock wave generating unit that receives the pulsed power and generates the pulsed shock waves, and a housing having a liquid and a drug disposed therein. The liquid is expanded by the pulsed shock waves, and pressure is applied to the drug to inject the drug.

### [ADVANTAGEOUS EFFECTS OF THE INVENTION]

According to an embodiment of the inventive concept, the needle-free injector using the pulsed shock waves may easily adjust the degree of expansion of the liquid (e.g., the rate of volume expansion by the gas generated in the liquid), may be implemented with small and economical equipment, and may prevent damage to an optical system.

In addition, microbubble generation and break-down formation may be sequentially performed by providing only a high voltage without needing to apply a low voltage for generating microbubbles and thereafter provide a high voltage for forming break-down, and thus there is an effect that control of the needle-free injector is simplified.

### [DESCRIPTION OF THE DRAWINGS]

FIG. 1A is a schematic sectional view illustrating a needle-free injector using pulse shock waves according to an embodiment of the inventive concept.
FIG. 1B is a schematic sectional view of region A of FIG. 1A taken in the -Z direction.
FIG. 2A is a schematic sectional view illustrating the needle-free injector using the pulsed shock waves according to an embodiment of the inventive concept.
FIG. 2B is a schematic sectional view of region A of FIG. 2A taken in the -Z direction.

### [BEST MODE]

The above and other aspects, features, and advantages of the inventive concept will become apparent from the following description of embodiments given in conjunction with the accompanying drawings. However, the inventive concept is not limited to the embodiments disclosed herein and may be implemented in various different forms. Herein, the embodiments are provided to provide complete disclosure of the inventive concept and to provide thorough understanding of the inventive concept to those skilled in the art to which the inventive concept pertains, and the scope of the inventive concept should be limited only by the accompanying claims and equivalents thereof.

Terms used herein are only for description of embodiments and are not intended to limit the inventive concept. As used herein, the singular forms are intended to include the plural forms as well, unless context clearly indicates otherwise. It will be further understood that the terms "comprise" and/or "comprising" specify the presence of stated features, components, and/or operations, but do not preclude the presence or addition of one or more other features, components, and/or operations. In addition, identical numerals will denote identical components throughout the specification, and the meaning of "and/or" includes each mentioned item and every combination of mentioned items. It will be understood that, although the terms first, second, etc. may be used herein to describe various components, these components should not be limited by these terms. These terms are only used to distinguish one component from another component. Thus, a first component discussed below could be termed a second component without departing from the teachings of the inventive concept.

Unless otherwise defined, all terms (including technical and scientific terms) used herein have the same meaning as commonly understood by those skilled in the art to which the inventive concept pertains. It will be further understood that terms, such as those defined in commonly used dictionaries, should not be interpreted in an idealized or overly formal sense unless expressly so defined herein.

Hereinafter, embodiments of the inventive concept will be described in detail with reference to the accompanying drawings.

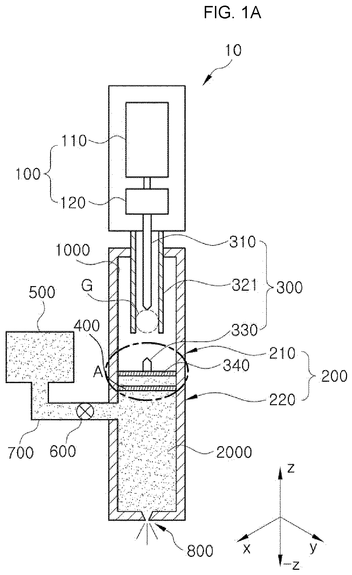

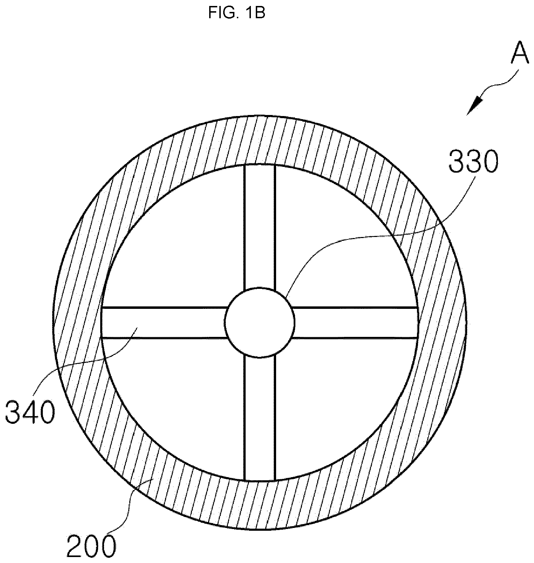

FIG. 1A is a schematic sectional view illustrating a needle-free injector using pulsed shock waves according to an embodiment of the inventive concept. FIG. 1B is a schematic sectional view of region A of FIG. 1A taken in the -Z direction.

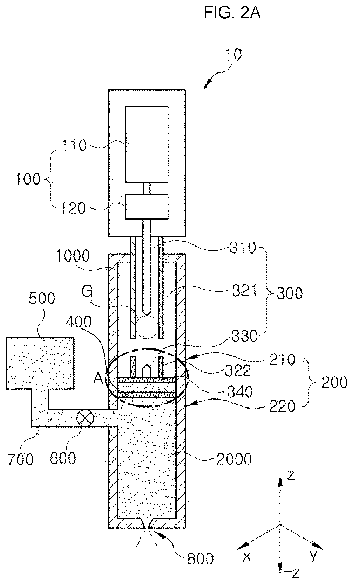

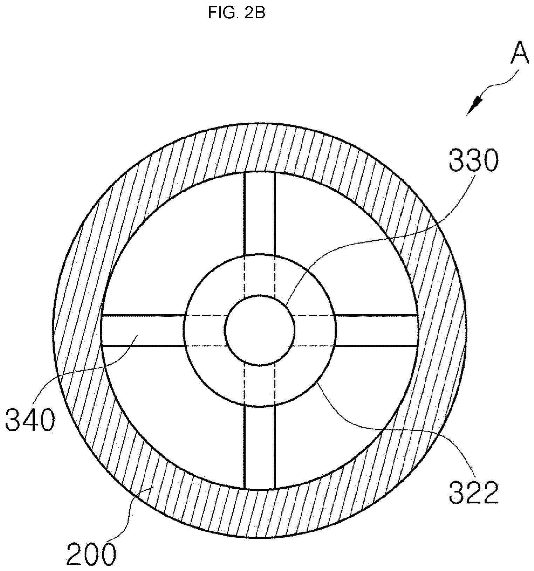

FIG. 2A is a schematic sectional view illustrating the needle-free injector using the pulsed shock waves according to an embodiment of the inventive concept. FIG. 2B is a schematic sectional view of region A of FIG. 2A taken in the -Z direction.

Referring to FIGS. 1A, 1B, 2A, and 2B, a needle-free injector 10 using the pulsed shock waves according to an embodiment of the inventive concept includes a power unit 100, a pulsed shock wave generating unit 300, and a housing 200. The housing 200 includes an upper housing 210 and a lower housing 220. The needle-free injector 10 using the pulsed shock waves according to an embodiment of the inventive concept includes a shock wave transmitting unit 400 and a n injection unit 800.

The power unit 100 instantaneously generates pulsed power by operating a voltage charged in a capacitor as a switch. Although not illustrated, for example, the power unit 100 includes a power supply unit. The power supply unit may preferably be a generator. The generator provides electricity for generating the pulsed power. For example, the generator may boost a low voltage to a high voltage and may generate the pulsed power through the switch.

The power unit 100 may include an electricity storage unit 110 and a switch 120. The electricity storage unit 110 may preferably be at least one selected from a capacitor and an inductor.

In addition, the power unit 100 may further include an electrical circuit that maintains the form of a generated pulse. In this case, the electrical circuit may preferably be a pulse forming network (PFN) and may prevent the form of a square pulse from collapsing due to parasitic inductance, thereby maintaining the form of the pulse.

Electricity generated by the power supply unit may be firstly charged in the electricity storage unit 110. When the switch 120 is turned on, the pulsed power charged in the electricity storage unit 110 may be transmitted to the pulsed shock wave generating unit 300. The switch 120 may supply or cut off electricity. For example, the switch 120 may adjust the rising time of pulsed shock waves by a user.

Specifically, the power unit 100 further includes a generator (not illustrated) for charging the electricity storage unit 110. The generator charges the electricity storage unit by converting an AC voltage into a DC voltage and providing a current to the electricity storage unit. Pulsed power under a specific condition is provided to the pulsed shock wave generating unit by adjusting the switch 120 after the electricity storage unit 110 is charged. That is, the switch 120 provides, to the pulsed shock wave generating unit 300, a voltage raised to a high voltage value within a short period of time (e.g., several microseconds) and maintained at a constant value.

The pulsed shock wave generating unit 300 receives the pulsed power and generates pulsed shock waves. The pulsed shock wave generating unit 300 is disposed in the upper housing 210. The pulsed shock wave generating unit 300 expands a liquid 1000 in the upper housing 210 by generating the pulsed shock waves. The expanded liquid 1000 moves the shock wave transmitting unit 400 in the direction from the upper housing 210 to the lower housing 220 and injects a drug 2000 through the injection unit 800.

The pulsed shock wave generating unit 300 generates the pulsed shock waves.

The pulsed shock wave generating unit 300 may include a cable and may be, for example, a coaxial cable. The cable may maintain low inductance by keeping a short current path. When the cable maintains the low inductance, it may be advantageous for fast pulse generation.

The pulsed shock wave generating unit 300 may include one or more shock wave generating electrodes and one or more insulating tubes. The one or more shock wave generating electrodes may receive the pulsed power so that a high voltage may be applied thereto.

The one or more shock wave generating electrodes may include, for example, a first shock wave generating electrode 310 and a second shock wave generating electrode 330, and although not illustrated, more shock wave generating electrodes may be included.

Hereinafter, it will be exemplified that one first shock wave generating electrode 310 and one second shock wave generating electrode 330 are provided. However, the inventive concept is not limited thereto, and a plurality of first shock wave generating electrodes 310 and/or a plurality of second shock wave generating electrodes 330 may be provided.

FIGS. 1A and 2A illustrate one example that the first shock wave generating electrode 310 is connected to the switch 120. However, without being limited thereto, the first shock wave generating electrode 310 may be connected to the switch 120 by a separate connecting unit. The connecting unit may be connected to the first shock wave generating electrode 310 and the second shock wave generating electrode 330, respectively, and may apply a voltage to allow a current to flow.

Insulating tubes 321 and 322 are adjacent to at least one of the shock wave generating electrodes 310 and 330. The insulating tubes 321 and 322 may or may not make contact with at least one of the shock wave generating electrodes 310 and 330. The insulating tubes 321 and 322 include the first insulating tube 321 and the second insulating tube 322.

The first shock wave generating electrode 310 may be disposed in the first insulating tube 321. The length of the first insulating tube 321 in the -Z direction may be longer than the length of the first shock wave generating electrode 310 in the -Z direction.

The first insulating tube 321 may have various shapes, such as a circular shape, a quadrilateral shape, and the like, when viewed from above, but is not limited thereto.

The first shock wave generating electrode 310 is inserted into the first insulating tube 321. One end of the first shock wave generating electrode 310 is not exposed outside the first insulating tube 321. More specifically, the one end of the first shock wave generating electrode 310 that is opposite one end of the second shock wave generating electrode 330 at the closest distance is not exposed outside the first insulating tube 321.

A shock wave generating unit G generates microbubbles for generation of pulsed shock waves as a current instantaneously flows between the shock wave generating electrodes 310 and 330. The shock wave generating unit G may mean, for example, a region between the first shock wave generating electrode 310 and the first insulating tube 321. The shock wave generating unit G may mean, for example, a region defined by the first shock wave generating electrode 310, the second shock wave generating electrode 330, and the first insulating tube 321.

The second shock wave generating electrode 330 may be connected to a cable 340. The second shock wave generating electrode and the cable may be connected in various manners

In an embodiment, the liquid 1000 may be disposed between the cable 340 and the shock wave transmitting unit 400. For example, water may be disposed between the cable 340 and the shock wave transmitting unit 400. The shock wave transmitting unit 400 may have the form of a film formed of various materials and, for example, may be an elastic film.

In another embodiment, the second shock wave generating electrode and the cable connected thereto may be coupled to the shock wave transmitting unit 400 having a film form and may be moved toward the lower housing together with a separation film (that is, the shock wave transmitting unit) by liquid expansion. In this case, when the shock wave transmitting unit expands toward the lower housing, only a peripheral region other than the center of the shock wave transmitting unit may be formed to have elasticity, and the second shock wave generating electrode may be disposed at the center of the shock wave transmitting unit. Furthermore, the cable coupled to the shock wave transmitting unit may be stretched together while the shock wave transmitting unit is expanded toward the lower housing by expansion of the upper housing, or may be broken when the shock wave transmitting unit is expanded and then may be short-circuited again when the shock wave transmitting unit is restored to a normal state.

Although not illustrated, the cable 340 may preferably be connected to the power unit 100.

Although not illustrated, the second shock wave generating electrode 330 may make contact with the shock wave transmitting unit 400.

Furthermore, the second shock wave generating electrode 330 may be disposed on one surface of the upper housing 210. In this case, the second shock wave generating electrode 330 may be disposed on the one surface of the upper housing 210 while being located under one end of the first insulating tube 321, that is, the shock wave generating unit G.

The second shock wave generating electrode 330 may be connected to the cable 340 without the second insulating tube 322, or may be disposed in the second insulating tube 322.

The second insulating tube 322 may have various shapes, such as a circular shape, a quadrilateral shape, and the like, when viewed from above, but is not limited thereto.

For example, referring to FIGS. 1A and 1B, the second shock wave generating electrode 330 may be connected to the cable 340 without being inserted into the second insulating tube (322 of FIGS. 2A and 2B).

For example, referring to FIGS. 2A and 2B, the second shock wave generating electrode 320 may be disposed in the second insulating tube 322. The length of the second insulating tube 322 in the +Z direction may be longer than the length of the second shock wave generating electrode 330 in the +Z direction.

When the second shock wave generating electrode 330 is inserted into the second insulating tube 322, the one end of the second shock wave generating electrode 330 is not exposed outside the second insulating tube 322. More specifically, the one end of the second shock wave generating electrode 330 that is opposite the one end of the first shock wave generating electrode 310 at the closest distance is not exposed outside the second insulating tube 322.

Referring again to FIGS. 1A, 1B, 2A, and 2B, as a specific example, the first shock wave generating electrode 310 inserted into the longer first insulating tube 321 extends in an up/down direction (that is, the Z-axis direction), and the second shock wave generating electrode 330 is disposed in an opposite direction. As the first shock wave generating electrode 310 extends long in the upper housing 210 (that is, a chamber filled with a liquid), the first shock wave generating electrode 310 may extend to a region adjacent to the shock wave transmitting unit 400 separating from the lower housing 220 . Furthermore, the second shock wave generating electrode 330 may be coupled to the shock wave transmitting unit 400. In addition, the first shock wave generating electrode 310 and the second shock wave generating electrode 330 are disposed in opposite directions at a specific distance at which a spark due to a plasma phenomenon is able to be generated when a high voltage is applied.

The pulsed shock wave generating unit 300 of an embodiment of the inventive concept may generate pulsed shock waves by a one-step voltage provision method of directly applying a high voltage for spark generation rather than an existing two-step voltage provision method of providing a low voltage for generating microbubbles and then providing a high voltage for spark generation. This is because when a high voltage is applied in the region of the first insulating tube 321 longer than the one end of the first shock wave generating electrode 310, microbubbles due to a temperature rise may be generated and accordingly, breakdown may occur to generate a spark. That is, a spark may be generated between the first insulating tube 321 longer than the one end of the first shock wave generating electrode 310 and the second shock wave generating electrode 330.

Specifically, in the space inside the first insulating tube 321 at the distal end of the first shock wave generating electrode 310, temperature rises depending on application of a high voltage, and a gas dissolved in the liquid thermally expands to generate microbubbles. As the microbubbles are generated (that is, cavitation occurs in the liquid) within a short period of time, a spark by a plasma phenomenon is generated due to the microbubbles between the first shock wave generating electrode 310 and the second shock wave generating electrode 330 and the applied high voltage, and internal expansion occurs in the upper housing 210.

The housing 200 has a sealed accommodation space. The liquid 1000 and the drug 2000 are disposed in the housing 200. The housing 200 may be divided into the upper housing 210 and the lower housing 220 by the shock wave transmitting unit 400.

The upper housing 210 has a sealed accommodation space. The liquid 1000 is disposed in the upper housing 210. The liquid 1000 may be, for example, water. That is, when the liquid is water, a gas may be dissolved so that microbubbles are able to be generated. However, without being limited thereto, the liquid 1000 may include various liquid materials such as polymer sol and gel, for example, alcohol or polyethylene glycol.

The upper housing 210 may have a schematically cylindrical shape. An upper end of the upper housing 210 may be connected to a transmitting unit. The shock wave transmitting unit 400 may be disposed at a lower end of the upper housing 210.

The volume of the liquid 1000 disposed in the upper housing 210 may be expanded by pulsed shock waves. When the volume of the liquid 1000 is increased by the pulsed shock waves, the pressure in the upper housing 210 is increased.

The lower housing 220 has a sealed accommodation space. The drug 2000 is disposed in the lower housing 220. The lower housing 220 may have a schematically cylindrical shape. The shock wave transmitting unit 400 may be disposed at an upper end of the lower housing 220. A lower end of the lower housing 220 may be connected to the injection unit 800. One side of the lower housing 220 may be connected to a drug delivery unit 700.

When the pressure in the upper housing 210 is increased, pressure is applied to the inside of the lower housing 220. That is, the pressure in the lower housing 220 may be increased. Accordingly, the pressure may be applied to the drug 2000. The drug 2000 may be injected through the injection unit 800 and then injected into a user. A detailed description thereof will be given below.

The shock wave transmitting unit 400 is provided between the upper housing 210 and the lower housing 220. The shock wave transmitting unit 400 divides the housing 200 into the upper housing 210 and the lower housing 220.

The shock wave transmitting unit 400 separates the upper housing 210 and the lower housing 220. One surface of the upper housing 210 and one surface of the lower housing 220 are formed by the shock wave transmitting unit 400. Accordingly, the expansion of the liquid 1000 disposed in the upper housing 210 may cause an increase in the pressure in the lower housing 220 through deformation of the shock wave transmitting unit 400.

The shock wave transmitting unit 400 is not changed in quality or damaged by pulsed shock waves. The shock wave transmitting unit 400 does not absorb pulsed shock waves and is vibrated by the pulsed shock waves. The shock wave transmitting unit 400 has elasticity. The shock wave transmitting unit 400 transmits only the pressure generated by the increase in the volume of the liquid 1000 to the inside of the lower housing 220. The shock wave transmitting unit 400 transmits only the pressure generated by the increase in the volume of the liquid 1000 to the drug 2000 in the lower housing 220. The shock wave transmitting unit 400 blocks penetration of the liquid 1000 and the drug 2000, heat transfer, and the like.

The shock wave transmitting unit 400 may be formed of, for example, natural rubber or synthetic rubber harmless to the human body.

Furthermore, when the shock wave transmitting unit 400 includes the second shock wave generating electrode 321, the second shock wave generating electrode 321 may be disposed at the center of the shock wave transmitting unit 400, and a conductive wire extending from the second shock wave generating electrode 321 may be included. As the region surrounding the second shock wave generating electrode 321 of the shock wave transmitting unit 400 has elasticity, it may be restored after being stretched by the increase in the pressure in the upper housing 210.

The injection unit 800 is disposed in the lower housing 220 as an injection nozzle. For example, the injection unit 800 may be defined in the form of a hole at the lower end of the lower housing 220. However, the inventive concept is not limited thereto, and if the injection unit 800 is able to inject the drug, the injection unit 800 may be connected to the lower housing 220 and may protrude in the direction from the upper end to the lower end of the lower housing 220. The injection unit 800 injects the drug 2000. The injection unit 800 may inject the drug 2000 in the Z-axis direction.

Furthermore, the speed at which the drug is injected is determined based on the diameter of the injection unit 800. That is, if the injection speed is low, the drug may not be injected into a skin, and therefore the injection unit 800 may be implemented to have a nozzle diameter by which the drug is injected at an appropriate speed based on the pressure transmitted from the upper housing 210 to the lower housing 220.

For example, the injection unit 800 may have a diameter of 50 micrometers to 1000 micrometers. When the diameter of the injection unit 800 is less than 50 micrometers, the amount of the injected drug 2000 may be less, and the drug 2000 may not be injected to a sufficient depth into the body of the user into which the drug 2000 is injected. When the diameter of the injection unit 800 exceeds 1000 micrometers, the injected microjet may have a large diameter, and therefore the amount of the drug 2000 bounced off the surface of the skin may be increased so that waste of the drug 2000 may become severe. The injection unit 800 may inject the drug 2000 in the Z-axis direction. In this specification, the "Z-axis direction" means the direction of an axis orthogonal to the X-axis direction (horizontal direction) and the Y-axis direction (vertical direction) in a three-dimensional coordinate system. More specifically, the injection unit 800 may inject the drug 200 in the direction from the upper housing 210 to the lower housing 220.

When the volume of the liquid 1000 is increased by the pulsed shock waves applied to the liquid 1000 as mentioned above, the pressure in the upper housing 210 is increased, and pressure is applied to the inside of the lower housing 220. Accordingly, the pressure may be applied to the drug 2000, and the pressurized drug 2000 may be injected into a userthrough the injection unit 800.

The needle-free injector 10 using the pulsed shock waves according to an embodiment of the inventive concept may further include a drug storage unit 500, the drug delivery unit 700, and a check valve 600.

The drug storage unit 500 stores the drug 2000 to be provided to the lower housing 220. For example, the drug storage unit 500 may be disposed on a side surface of the lower housing 220.

The drug delivery unit 700 receives the drug 2000 from the drug storage unit 500 and provides the drug 2000 to the lower housing 220. For example, the drug delivery unit 700 may be connected to a side surface of the lower housing 220.

The check valve 600 allows the drug 2000 to be delivered only in the direction from the drug storage unit 500 to the lower housing 220. For example, the check valve 600 prevents the drug 2000 from being delivered in the direction from the lower housing 220 to the drug storage unit 500. For example, the check valve 600 may be disposed in the drug delivery unit 700.

Furthermore, a needle-free injector 10 using pulse shock waves Furthermore, the needle-free injector 10 using pulsed shock waves according to another embodiment of the inventive concept further includes a liquid circulation unit (not illustrated). The liquid circulation unit serves to circulate a liquid in an upper housing. As microbubbles are generated and a spark due to a plasma phenomenon is generated, the amount of gas dissolved in the liquid may be decreased, and the pressure in the upper housing 210 may be increased by the generated gas. Accordingly, the liquid circulation unit may circulate the liquid in the upper housing 210 to fill the upper housing 210 with the liquid capable of generating appropriate pressure. Thus, drug injection of the needle-free injector 10 may be constantly performed.

Specifically, the liquid circulation unit may include a solenoid valve and may circulate and change the liquid in the upper housing 210 by opening the solenoid valve as needed.

Moreover, the needle-free injector 10 using pulsed shock waves according to another embodiment of the inventive concept further includes a pressure sensor (not illustrated). The pressure sensor serves to measure the pressure before and after a spark is generated and when the spark is generated. To this end, the pressure sensor may be disposed at a specific position in the upper housing 210.

For example, the pressure sensor may detect that the pressure in the upper housing 210 is raised to a reference value or more and may circulate a liquid in the upper housing 210 by driving a liquid circulation unit such that the shock wave transmitting unit 400 is in an equilibrium state. In addition, the pressure sensor measures the pressure generated when the spark is generated, and when the measurement value of the pressure sensor is not greater than or equal to the reference value during operation, a controller (not illustrated) determines that the gas dissolved in the liquid is too little and circulates the liquid in the upper housing 210.

Hereinafter, a method of injecting the drug 2000 to a user by using the needle-free injector 10 using the pulsed shock waves according to an embodiment of the inventive concept will be described in brief.

When the power unit 100 generates pulsed power, the pulsed shock wave generating unit 300 receives the pulsed power and generates pulsed shock waves. When the pulsed shock waves are generated, the volume of the liquid 1000 provided in the upper housing 210 is expanded. As the volume of the liquid 1000 is expanded, the pressure in the upper housing 210 is increased. As the pressure in the upper housing 210 is increased, the shock wave transmitting unit 400, which has elasticity, transmits the increased pressure into the lower housing 220. At this time, the shock wave transmitting unit 400 is not damaged by the pressure. When the increased pressure in the upper housing 210 is provided to the inside of the lower housing 220, the drug 2000 may be injected into a user through the injection unit 800. When the drug 2000 is additionally required in the lower housing 220, the check valve 600 may be opened, and the drug 2000 may be provided from the drug storage unit 500 into the lower housing 220.

The needle-free injector 10 using the pulsed shock waves according to an embodiment of the inventive concept may adjust the rising time from nanoseconds to milliseconds through the power unit 100 that generates the pulsed power and thus may generate short shock waves. Due to this, the liquid may be thermally expanded within a short period of time, and the drug may be injected into a user at high speed.

Further, the needle-free injector 10 may adjust the injection amount of the drug in the lower housing 220 by adjusting the intensity of the pressure generated in the upper housing 210 by the generated pulsed power. Accordingly, a user may inject the drug in a desired amount, and thus the drug may be prevented from being wasted.

Furthermore, by implementing an electric shock wave needle-free injector that solves the problem in which a low voltage has to be first provided to generate microbubbles, drug injection may be more rapidly performed.

Moreover, the needle-free injector 10 using the pulsed shock waves according to an embodiment of the inventive concept uses pulsed power rather than a laser. Accordingly, a problem occurring when the laser is used, more specifically, a large device structure and expensive facility cost are not required. In addition, since an optical part through which a laser beam passes is not required, the needle-free injector 10 may fundamentally solve a problem caused by the optical part, for example, a problem in which a cable arrangement state between a main body and a needle-free injector has to be limited to accurately transmit a laser from the main body generating the laser to the needle-free injector (e.g., a handpiece unit injecting a drug).

While the inventive concept has been described with reference to embodiments, it will be apparent to those skilled in the art that various changes and modifications may be made without departing from the spirit and scope of the inventive concept. Therefore, it should be understood that the above embodiments are not limiting, but illustrative.

## Claims

1. A needle-free injector using pulsed shock waves, the needle-free injector comprising:
a power unit configured to generate pulsed power;
a pulsed shock wave generating unit configured to receive the pulsed power and generate the pulsed shock waves;
an upper housing in which a liquid and the pulsed shock wave generating unit are disposed therein;
a lower housing connected to the upper housing and having a drug disposed therein;
a shock wave transmitting unit provided between the upper housing and the lower housing and configured to transmit the shock waves generated in the upper housing to the lower housing; and
a n injection unit disposed in the lower housing and configured to inject the drug.

2. The needle-free injector of claim 1, wherein the power unit includes:
a power supply unit configured to supply a voltage and a current;
an electricity storage unit configured to store electricity supplied from the power supply unit; and
a switch configured to apply stored electrical energy as pulsed power from the electricity storage unit.

3. The needle-free injector of claim 2, wherein the power unit further includes an electrical circuit configured to maintain a form of a generated pulse.

4. The needle-free injector of claim 1, wherein the pulsed shock wave generating unit includes:
one or more shock wave generating electrodes configured to receive the pulsed power and allow a current to flow; and
an insulating tube disposed in proximity to at least one of the shock wave generating electrodes in contact or non-contact with the at least one of the shock wave generating electrodes.

5. The needle-free injector of claim 4, wherein in the pulsed shock wave generating unit, one end of the electrode is not exposed outside the insulating tube.

6. The needle-free injector of claim 4, wherein the shock wave generating electrodes include a first shock wave generating electrode and a second shock wave generating electrode, and
wherein the insulating tube includes a first insulating tube having the first shock wave generating electrode located therein and longer than the first shock wave generating electrode.

7. The needle-free injector of claim 6, wherein the insulating tube further includes a second insulating tube having the second shock wave generating electrode located therein and longer than the second shock wave generating electrode.

8. The needle-free injector of claim 1, wherein the pulsed shock wave generating unit includes a cable configured to connect the power unit and a shock wave generating electrode.

9. The needle-free injector of claim 1, wherein when bubbles generated from the pulsed shock wave generating unit increase pressure in the upper housing, the shock wave transmitting unit transmits the increased pressure to the lower housing.

10. The needle-free injector of claim 1, wherein the injection unit injects the drug when increased pressure in the upper housing is transmitted to the lower housing.

11. The needle-free injector of claim 1, further comprising:
a drug storage unit configured to store the drug to be provided to the lower housing; and
a check valve configured to allow the drug to be delivered only in a direction from the drug storage unit to the lower housing.
